# EUROPEAN PATENT APPLICATION

(11) **EP 2 796 204 A1**
(43) Date of publication of application: **29.10.2014**
(21) Application number: 12858866.2
(22) Date of filing: 20.11.2012
(51) Int. Cl.: B05B 1/02, B05B 1/12, A61L 9/14

(54) **SPRAYER**

(30) Priority: 20.12.2011 KR 20110137845
(71) Applicant: Jeon, Eun Soo, Seoul 157-280 (KR); Baek, Hye Sun, Incheon 405-825 (KR)
(72) Inventor: Jeon, Eun Soo, Seoul 157-280 (KR); Baek, Hye Sun, Incheon 405-825 (KR)
(74) Representative: De Clercq, Ann G. Y.
(86) International application number: PCT/KR2012/009840
(87) International publication number: WO 2013/094884

(57) **Abstract**

The present invention relates to a sprayer. The sprayer according to the present invention comprises: a vessel main body where an air intake port and a mist discharge port are formed; a spray nozzle which has an air inlet and a liquid inlet, and which is disposed inside the vessel main body; and an atomization member which collides with or diverts mist sprayed through the spray nozzle. The present invention is capable of effectively atomizing a disinfectant, pesticide, or an environment improving agent such as an air freshener or a deodorant agent in a fine particle state, and is thus economically feasible and environmentally friendly since the possibility of pollution can be reduced.

## Description

### BACKGROUND OF THE INVENTION

### TECHNICAL FIELD

The present invention relates to a sprayer, and more particularly, to a sprayer that is capable of effectively atomizing a disinfectant or agricultural chemicals such as an anti-virus agent, an anti-bacterial agent, an anti-fungal agent, a pesticide and the like or an environment improving agent such as a fragrant, a deodorant agent and the like, to macro-particle sizes, and in large quantities spraying the atomized mist, so that the mist can be sprayed in a relatively small quantity upon the work for the prevention of infectious diseases or for the environment improvement, thus reducing the possibility of the pollution of soil or dwelling environment and further, the disinfectant or environment improving agent can be uniformly permeated and accessed to an object to be disinfected through the atomization, thus enhancing the work efficiency.

### BACKGROUND ART

Generally, sprayers are classified in various ways, but in accordance with their atomization ways, they are divided into a hydraulic sprayer, an airflow sprayer, a rotary sprayer, and an ultrasonic sprayer. In this case, the hydraulic sprayer pressurizes a liquid like water to a high pressure and atomizes the pressurized liquid together air through a nozzle, the airflow sprayer passes high pressure gas at a fast flow rate, induces a pressure-reduced state to a branch point, mixes a liquid and air through suction, and atomizes the mixture through a nozzle. Further, the rotary sprayer passes a high pressure liquid through a rotor on which fine slits are formed and atomizes the liquid, and the ultrasonic sprayer atomizes a fluid passing through an ultrasonic transducer or a plurality of baffles on which fine slits are formed by using ultrasound.

Additionally, a compression sprayer is provided wherein compressive gas is put into a vessel and a liquid is pressurized through the expansion force of the compressive gas, thus spraying the mixture made by mixing the compressive gas and the liquid, a mister is provided wherein fuel is ignited and exploded in an evaporator and the exhaust gas is discharged through an exhaust pipe, thus atomizing a liquid by means of exhaust pressure, and an evaporation sprayer is provided wherein a liquid is evaporated and gasified through a heating coil.

However, the hydraulic sprayer generally makes use of an internal combustion engine for operating a plunger and further needs a compression controlling mechanism and a pressure tank, thus increasing the size and weight of the product and making it hard to provide economical advantages.

Further, the rotary sprayer and the ultrasonic sprayer are low in weight and compact in configuration, but generally, since disinfectant or pesticide is toxic and corrosive, it is hard to clean the fine slit rotor, ultrasonic transducer, or the baffles on which the fine slits are formed to form ultrasound. Furthermore, they will be likely to be corrosive.

The airflow sprayer includes a compressor, a motor, a liquid storage tank and a nozzle and has various sizes and weights from large-sized one to compact small-sized one. However, the mean diameters of the macro particles in the range between 20 µm and 200 µm through atomization are substantially large, so that the disinfectant or the environment improving agent is used in relatively large quantities, thus failing to provide economical advantages and causing soil or dwelling environment to be polluted and poisoned through the use of the excessive quantity. Besides, the atomization is not gently conducted, so that the disinfectant or environment improving agent cannot be uniformly permeated and accessed to an object to be disinfected through the atomization, thus providing low work efficiency.

In more detail, since the use of Freon has been recently prohibited, the compression sprayer makes use of compressive gas like propane gas having high inflammability, thus providing low safety and further, the compression sprayer has to use a disposable container, thus failing to provide economical advantages. The mister may cause air contamination due to the exhaust gas, and the evaporation sprayer has a relatively low efficiency.

So as to solve the problems as mentioned above, accordingly, there is a definite need for the development of a new sprayer capable of providing excellent work efficiency ability, reliability and economical advantages.

### DISCLOSURE

### TECHNICAL PROBLEM

Accordingly, the present invention has been made in view of the above-mentioned problems occurring in the prior art, and it is an object of the present invention to provide a sprayer that is capable of effectively atomizing a disinfectant or agricultural chemicals such as an anti-virus agent, an anti-bacterial agent, an anti-fungal agent, a pesticide and the like or an environment improving agent such as a fragrant, a deodorant agent and the like, to macro-particle sizes.

It is another object of the present invention to provide a sprayer that is capable of having a relatively large sprayed area even if a disinfectant, agricultural chemicals or environment improving agent is used in relatively small quantities, thus providing many economical advantages.

It is still another object of the present invention to provide a sprayer that is capable of using a relatively small quantity of a disinfectant, agricultural chemicals or environment improving agent upon the work for the prevention of infectious diseases or for the environment improvement, thus reducing the possibility of the pollution of soil or dwelling environment.

It is still yet another object of the present invention to provide a sprayer that is capable of allowing a disinfectant, agricultural chemicals or environment improving agent to be uniformly penetrated and accessed to an object to be disinfected through the atomization.

It is yet another object of the present invention to provide a sprayer that is capable of being portable and compact in size, thus enhancing work efficiency.

It is still another object of the present invention to provide a sprayer that is capable of adjusting the sizes of the macro particles and the quantity of sprayed mist in simple and easy manner in a working site.

### TECHNICAL SOLUTION

To achieve the above objects, a sprayer according to the present invention adopts an round plate, roller, or sphere-shaped atomization member that is spaced apart from a spray nozzle by a given distance, the spaced distance between the atomization member and the spray nozzle being selectively adjustable, so that the mist sprayed from the spray nozzle collides against the atomization member, atomized and then divertedly sprayed.

### ADVANTAGEOUS EFFECT

As mentioned above, the sprayer of the present invention is capable of effectively atomizing a disinfectant or agricultural chemicals such as an anti-virus agent, an anti-bacterial agent, an anti-fungal agent, a pesticide and the like or an environment improving agent such as a fragrant, a deodorant agent and the like to macro-particle sizes, having a relatively large sprayed area even if a disinfectant, agricultural chemicals or environment improving agent is used in relatively small quantities, thus providing many economical advantages, using a relatively small quantity of the disinfectant, agricultural chemicals or environment improving agent upon the work for the prevention of infectious diseases or for the environment improvement, thus reducing the possibility of the pollution of soil or dwelling environment and being environmentally friendly, allowing the disinfectant, agricultural chemicals or environment improving agent to be uniformly penetrated and accessed to an object to be disinfected through the atomization, being portable and compact in size, thus enhancing work efficiency, and adjusting the sizes of the macro particles and the quantity of sprayed mist in simple and easy manner in a working site.

### BRIEF DESCRIPTION OF THE DRAWINGS

The above objects, other features and advantages of the present invention will become more apparent by describing the preferred embodiments thereof with reference to the accompanying drawings, in which:
FIGS.1a to 1c are perspective views showing the inside of a sprayer according to a first embodiment of the present invention.
FIGS.2a to 2d are perspective views showing a sprayer according to a second embodiment of the present invention, wherein FIGS.2a and 2b show the inside of the sprayer, FIG.2c shows the operating state of the sprayer, and FIG.2d shows a variation example of the sprayer according to the second embodiment of the present invention.
FIG.3 is a perspective view showing the inside of a sprayer according to a third embodiment of the present invention.
FIGS.4a and 4b are perspective views showing the inside of a sprayer according to a fourth embodiment of the present invention and the sprayer as a product.
FIGS.5a to 5c are perspective views showing a sprayer according to a fifth embodiment of the present invention, wherein FIG.5a shows the inside of the sprayer, FIG.5b shows the operating state of the sprayer, and FIG.5c shows the operating state of the sprayer where mist guides are mounted.
FIG.6 is a perspective view showing the separated state of a motor and a compressor from the sprayer.
FIGS.7a and 7b are perspective view showing the comparison between micro-particle sizes atomized by the sprayer according to the present invention and one conventional sprayer.

### Preferred Embodiments of the Invention

To achieve the first to fifth objects as mentioned above, according to first to fourth embodiments of the present invention, a sprayer includes: a main body where an air intake port and a mist discharge port are formed; a spray nozzle disposed inside the main body and having an air inlet and a liquid inlet; and an atomization member adapted to collide the mist sprayed through the spray nozzle thereagainst and to divert the direction of the mist.

To achieve the first to fifth objects as mentioned above, according to the first to fourth embodiments of the present invention, the sprayer has the atomization member adjustable in the spaced distance from the spray nozzle so as to control the particle sizes of the mist.

To achieve the first to fifth objects as mentioned above, according to the first embodiment of the present invention, the sprayer is configured wherein the atomization member has a shape of a round plate in such a manner as to be fixed to a top surface plate communicating with a mist discharge pipe on which the mist discharge port is formed by means of a plurality of stoppers, so that the outer peripheral surface of the inner peripheral wall of the main body and the outer peripheral surface of the atomization member are spaced preferably 2 to 20 mm apart from each other, more preferably 5 to 15 mm apart from each other, and most preferably 8 to 12 mm apart from each other and further, the spaced distances between the spray nozzle and the atomization member and between the atomization member and the top surface plate are preferably in the range between 10 mm and 100 mm, more preferably in the range between 15 mm and 90 mm, and most preferably in the range between 20 mm and 80 mm.

To achieve the sixth object as mentioned above, according to the first embodiment of the present invention, the sprayer is configured wherein the mist discharge pipe has a support rod disposed to traverse the bottom end opening thereof, the support rod having a screw hole formed at the center thereof, and a height adjustment bolt passed through the screw hole and coupled to the atomization member, so that the vertical distance between the spray nozzle and the atomization member is adjustable to the range between 10 mm and 100 mm.

To achieve the first to fifth objects as mentioned above, according to the first to fourth embodiments of the present invention, the spray nozzle is located on a supporter and includes a head having a liquid spray part and a plurality of air spray parts and a body having the air inlet and the liquid inlet, the supporter being formed of a plate having a plurality of support posts.

To achieve the first to fifth objects as mentioned above, according to the second embodiment of the present invention, the sprayer is configured to have a shoulder and a neck having a mist discharge port mounted on the main body and to have the atomization member having a shape of a roller, so that the ratio of the diameter of the main body to the diameter of the roller as the atomization member is in the range between 1 : 0.25 ∼ 0.45, and the vertical distance between a spray nozzle and the underside surface of the atomization member is preferably in the range between 10 mm and 100 mm, more preferably in the range between 15 mm and 90 mm, and most preferably in the range between 20 mm and 80 mm.

To achieve the sixth object as mentioned above, according to the second embodiment of the present invention, the sprayer is configured wherein the spray nozzle is located on the supporter and includes a head having a liquid spray part and a plurality of air spray parts and a body having an air inlet and a liquid inlet, the supporter being formed of a plate having a plurality of support posts disposed on the underside thereof, the plate having a pair of support frames erected on the top surface thereof in such a manner as to face each other, each support frame having a vertical slot formed thereon, and wherein a lever to which a cam is fitted is located passed through the pair of support frames and the main body, and as the cam rotates through the rotation of the lever, the roller-shaped atomization member moves upward and downward along the vertical slots, so that the vertical distance between the spray nozzle and the underside surface of the atomization member is adjustable to the range between 10 mm and 100 mm.

To achieve the first to fifth objects as mentioned above, according to the third embodiment of the present invention, a sprayer is configured to have a shoulder and a neck having a mist discharge port mounted on the main body and to have the atomization member having a shape of a sphere, so that through a plurality of spacers, the outer peripheral surface of the upper portion of the atomization member and the inner peripheral surface of the shoulder are spaced preferably 2 to 20 mm apart from each other, more preferably 5 to 15 mm apart from each other, and most preferably 8 to 12 mm apart from each other and further, the vertical distance between the spray nozzle and the underside surface of the atomization member is preferably in the range between 10 mm and 100 mm, more preferably in the range between 15 mm and 90 mm, and most preferably in the range between 20 mm and 80 mm.

To achieve the first to fifth objects as mentioned above, according to the fourth embodiment of the present invention, the sprayer is configured to have the atomization member having a shape of a disc formed convexedly on the upper and lower portions thereof and fixed to a top surface plate communicating with a mist discharge pipe on which the mist discharge port is formed by means of a plurality of stoppers, so that the outer peripheral surface of the inner peripheral wall of the main body and the outer peripheral surface of the atomization member are spaced preferably 2 to 20 mm apart from each other, more preferably 5 to 15 mm apart from each other, and most preferably 8 to 12 mm apart from each other and further, the vertical distances between the spray nozzle and the protruded lower surface of the atomization member and between the top surface plate and the protruded upper surface of the atomization member are preferably in the range between 10 mm and 100 mm, more preferably in the range between 15 mm and 90 mm, and most preferably in the range between 20 mm and 80 mm, wherein a plurality of intake ports is formed on the top surface plate and the spray nozzles are equally spaced apart from each other.

To achieve the sixth object as mentioned above, according to the fourth embodiment of the present invention, the sprayer is configured wherein a mist discharge pipe on which the mist discharge port is formed has a support rod disposed to traverse the bottom end opening thereof, the support rod having a screw hole formed at the center thereof, and a height adjustment bolt passed through the screw hole and coupled to the atomization member, so that the vertical distance between the spray nozzle and the protruded lower surface of the atomization member is adjustable to the range between 10 mm and 100 mm.

To achieve the first to sixth objects as mentioned above, according to the fourth embodiment of the present invention, the sprayer is configured wherein the spray nozzles are located on the supporter and each includes a head having a liquid spray part and a plurality of air spray parts and a body having an air inlet and a liquid inlet, the supporter being formed of a plate having a plurality of support posts disposed on the underside thereof, the plate having a plurality of slots formed thereon toward the center thereof, so that the plurality of spray nozzles is varied in position along the plurality of slots.

To achieve the first to sixth objects as mentioned above, according to the fourth embodiment of the present invention, the sprayer is configured wherein the plate has a plurality of through holes formed thereon.

To achieve the first to sixth objects as mentioned above, according to the fifth embodiment of the present invention, a sprayer includes: a plurality of sprayers each having a main body where an air intake port and a mist discharge port are formed, a spray nozzle disposed inside the main body and having an air inlet and a liquid inlet, and an atomization member adapted to collide the mist sprayed through the spray nozzle thereagainst and to divert the direction of the mist, the atomization member being adjustable in the spaced distance from the spray nozzle so as to control the particle sizes of the mist; a box fixedly containing the plurality of sprayers thereinto; and a plurality of intake pipes formed to communicate with the outside of the box, wherein the plurality of mist discharge ports is formed to communicate with the top surface of the box and a plurality of air pipes of the spray nozzles is extended outward from the box.

To achieve the first to sixth objects as mentioned above, according to the fifth embodiment of the present invention, the sprayer is configured wherein mist guides are mounted on the mist discharge ports.

To achieve the first to sixth objects as mentioned above, according to the fifth embodiment of the present invention, the sprayer is configured wherein each mist guide has a guide nozzle mounted on the front end periphery thereof.

According to the first to fifth embodiments of the present invention, a compressor, which is driven by a motor, is connected to the air pipe communicating with the air inlet port of the spray nozzle.

A term "micro-particles" used in the present invention refers to fine liquid drops having preferably mean particle diameter between 1 µm and 25 µm, more preferably between 1 µm and 8 µm, and most preferably between 2.4 µm and 4 µm formed by the atomization of an arbitrary solvent such as a water solution or alcohol made by dissolving or suspending a disinfectant or agricultural chemicals such as an anti-virus agent, an anti-bacterial agent, an anti-fungal agent, a pesticide and the like or an environment improving agent such as a fragrant, a deodorant agent and the like.

Further, a term "atomization" used in the present invention means the formation of mist through fog-like state through uniform distribution of a large number of micro-particle liquid drops.

Hereinafter, an explanation on a sprayer according to preferred embodiments of the present invention will be in detail given with reference to the attached drawings.

First, sprayers 1, 1a, 1b, 1c and 1d according to first to fifth embodiments of the present invention have a fundamental structure wherein a main body 10 wherein an air intake port 11a and a mist discharge port 15a or 16a are formed, the main body 10 having a lower portion in which a liquid 90 is stored; a spray nozzle 20 disposed inside the main body 10 and having an air inlet 23 and a liquid inlet 25; and an atomization member 30 adapted to collide the mist sprayed through the spray nozzle 20 thereagainst and to divert the direction of the mist.

The atomization member 30 may have various shapes such as a round plate, a roller, a sphere, a disc having protruded upper and lower portions, and the like, as will be discussed later, and may be made of stainless steel or synthetic resin on which inactive fluorine resin like Teflon is surface-coated or not surface-coated.

The atomization member 30 serves to primarily collide the mist having a large mean particle diameter between 20 µm and 100 µm discharged from the general air flowing spray nozzle 20 thereagainst and next divert the mist to allow the diverted mist to collide sequentially with the main body 10 and a top surface plate 13 or a shoulder 14, thus making the mist micro-particularized.

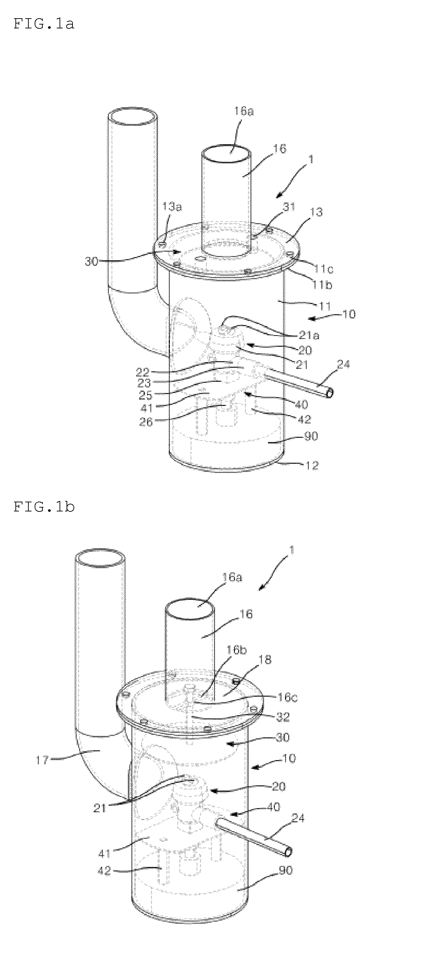

FIG.1a is a perspective view showing the inside of a sprayer according to a first embodiment of the present invention. As shown, the sprayer 1 includes the main body 10 having a side peripheral wall 11 having a top end flange 11b on which a plurality of fixing holes 11c is formed and the air intake port 11a formed on a given portion thereof, a bottom 12, and the top surface plate 13 having a mist discharge pipe 16 having the mist discharge port 16a erectly formed on the center of the top surface thereof and a plurality of fixing holes 13a formed on the area adjacent to the outer peripheral portion thereof; the round plate-shaped atomization member 30 fixed to the top surface plate 13 communicating with the mist discharge pipe 16 by means of a plurality of stoppers 31; the spray nozzle 20 disposed under the atomization member 30; and a supporter 40 adapted to support the spray nozzle 20.

The fixing holes 11c of the top end flange 11b of the side peripheral wall 11 and the fixing holes 13a of the top surface plate 13 are coupled to each other by means of appropriate fastening means like screw-coupling, but they are not limited to the above-mentioned coupling manner. That is, they may be coupled to each other by means of well-known means like welding.

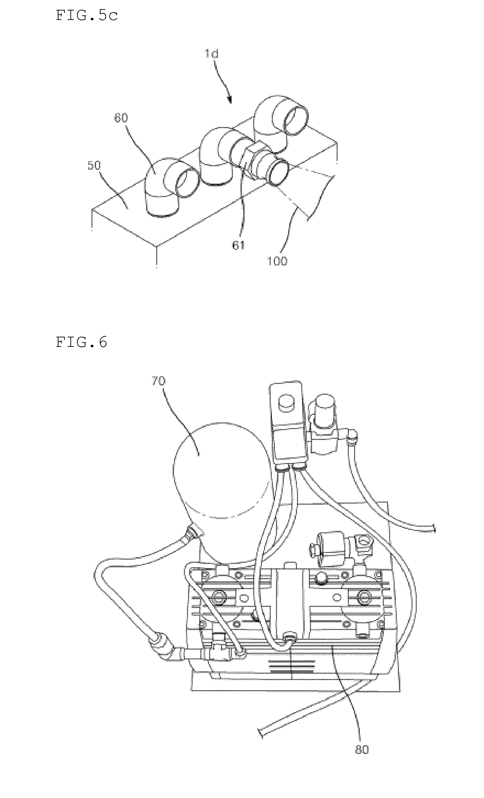

According to the first embodiment of the present invention, the spray nozzle 20 includes a head 21 having a liquid spray part 21b and a plurality of air spray parts 21a and a body 22 having the air inlet 23 and the liquid inlet 25, and as shown, the two air spray parts 21a are disposed to cross each other on the center of the single liquid spray part 21b to form turbulent flows. The air inlet 23 is connected to an air pipe 24 in such a manner as to be extended outward from the side peripheral wall 11 of the main body 10 and thus connected to an air compressor (see a reference numeral 70 in FIG.6), and the liquid inlet 25 is connected to a liquid pipe 26 through which the liquid 90 stored in the lower portion of the main body 10 is conveyed, the liquid 90 being made by dissolving or suspending disinfectant, agricultural chemicals or environment improving agent.

Next, the supporter 40 is formed of a plate 41 having a plurality of support posts 42, and the space above the plate 41 communicates with the space under the plate 41.

As the air compressor 79 connected to the outside is operated, the pressurized air through the air pipe 24 and the air inlet 23 is ejected at the crossing angle to each other from the plurality of air spray parts 21a formed on the head 21 of the spray nozzle 20, and accordingly, a reduced-pressure state is formed above the liquid inlet 25 formed on the head 21 of the spray nozzle 20, so that the liquid 90 is sprayed from the liquid inlet 25 by means of atmospheric pressure and strongly mixed with the air sprayed from the plurality of air spray parts 21a, thus forming mist having relatively large macro particle sizes in such a manner as to be sprayed upward and to strongly collide against the atomization member 30.

At this time, the outer peripheral surface of the inner peripheral wall of the main body 10 and the outer peripheral surface of the atomization member 30 are spaced preferably 2 to 20 mm apart from each other, more preferably 5 to 15 mm apart from each other, and most preferably 8 to 12 mm apart from each other, so that a given portion of the colliding mist falls downward but most of the colliding mist is kept colliding with the inner peripheral wall of the main body 10 through the spaced portion. Accordingly, the mist becomes more atomized, thus being discharged to the outside through the mist discharge port 16a of the mist discharge pipe 16.

In this case, if the spaced distance is less than 2 mm, undesirably, a quantity of discharged mist is seriously reduced, and contrarily, if more than 20 mm, undesirably, the atomization effect is not sufficiently generated.

At this time, the spaced distances between the spray nozzle 20 and the atomization member 30 and between the atomization member 30 and the top surface plate 13 are preferably in the range between 10 mm and 100 mm, more preferably between 15 mm and 90 mm, and most preferably between 20 mm and 80 mm. In this case, if the spaced distance is less than 10 mm, undesirably, a quantity of discharged mist is seriously reduced due to the increment of rear side drop quantity, and contrarily, if more than 100 mm, undesirably, the atomization effect is insufficiently generated.

In the drawings, a reference numeral 17 not explained yet indicates an intake pipe adapted to suck external air corresponding to the quantity of mist discharged, thus making it possible to keep gentle discharging.

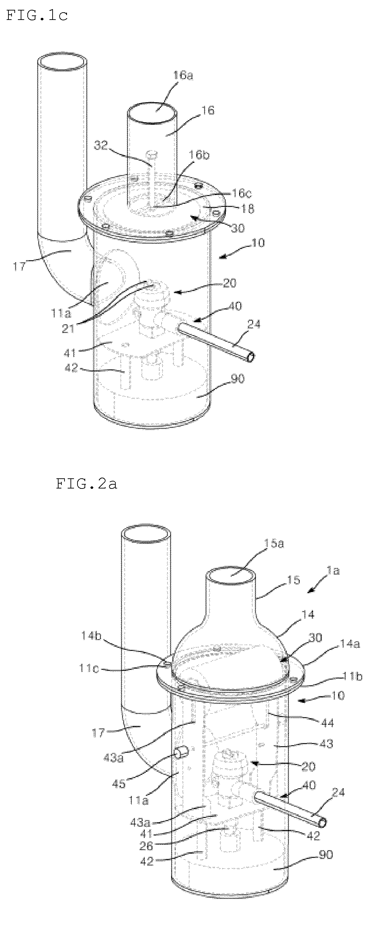

FIGS.1b and 1c are perspective views showing the inside of a variation sprayer according to the first embodiment of the present invention, wherein the atomization member 30 is adjustable in height. The variation sprayer 1 has the same configuration as that of FIG.1a, except that height adjustment means is applied to the atomization member 30, and accordingly, an explanation on the difference will be given for the brevity of the description.

As shown in FIGS.1b and 1c, the mist discharge pipe 16 has a support rod 16b disposed to traverse the bottom end opening thereof (to which a reference numeral is not designated), and the support rod 16b has a screw hole 16c formed at the center thereof. A height adjustment bolt 32 is passed through the screw hole 16c and coupled to the round plate-shaped atomization member 30, so that the vertical distance between the spray nozzle 20 and the atomization member 30 is adjustable to the range between 10 mm and 100 mm.

As shown in FIG.1b, the atomization member 30 is moved downward by the rotation of the height adjustment bolt 32 in such a manner as to be close to the spray nozzle 20, so that the degree of atomization becomes increased and contrarily, the quantity of discharge becomes decreased. On the other hand, as shown in FIG.1c, the atomization member 30 is moved upward by the rotation of the height adjustment bolt 32 in such a manner as to be spaced apart from the spray nozzle 20 to a maximum extent, so that the degree of atomization becomes decreased and contrarily, the quantity of discharge becomes increased.

The spaced distance range and other structures and functions have been mentioned above, and therefore, an explanation on them will be avoided for the brevity of the description.

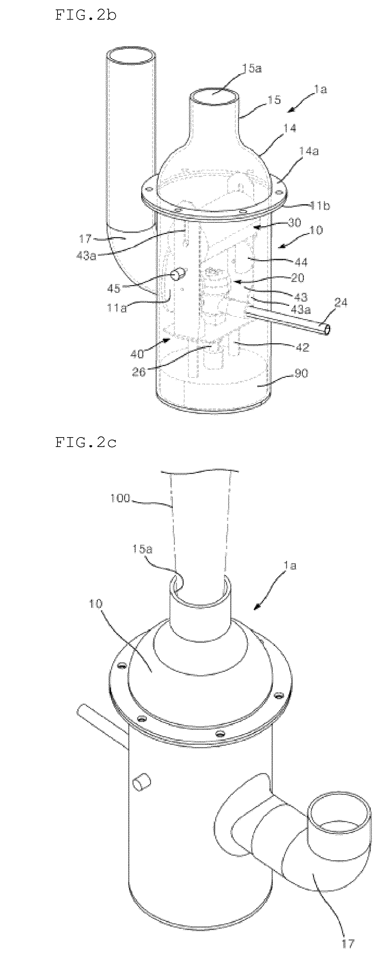

FIGS.2a and 2b are perspective views showing the inside of the sprayer 1a according to the second embodiment of the present invention.

As shown in FIGS.2a and 2b, the sprayer 1a according to the second embodiment of the present invention has the same configuration as the sprayer 1 according to the first embodiment of the present invention, except that a shoulder 14 and a neck 15 having a mist discharge port 15a are mounted on the main body 10 and the atomization member 30 has a shape of a roller. Accordingly, an explanation on the difference will be given for the brevity of the description.

In this case, the ratio of the diameter of the main body 10 to the diameter of the roller as the atomization member 30 is in the range between 1 : 0.25 ∼ 0.45. In this case, if the ratio is less than 0.25, undesirably, the atomization effect is insufficiently generated, and contrarily, if more than 0.45, undesirably, a quantity of discharged mist is seriously reduced due to the increment of rear side drop quantity.

Further, the vertical distance between the spray nozzle 20 and the underside surface of the atomization member 30 is preferably in the range between 10 mm and 100 mm, more preferably in the range between 15 mm and 90 mm, and most preferably in the range between 20 mm and 80 mm. According to the second embodiment of the present invention, the shoulder 14 becomes narrow as it goes upward, thus having a relatively large space portion, so that the spaced distance from the top portion of the atomization member 30 does not matter.

The atomization member 30 is adjustable up and down in height, but of course, it can be fixedly disposed within the spirit of the present invention.

As shown in FIGS.2a and 2b, in the same manner as in FIG.1a, the spray nozzle 20 is located on the supporter 40 and includes a head 21 having a liquid spray part 21b and two air spray parts 21a and a body 22 having an air inlet 22 and a liquid inlet 25. The supporter 40 has a plate 41 having a plurality of support posts 42 disposed on the underside thereof.

On the other hand, the plate 41 further has a pair of support frames 43 erected on the top surface thereof in such a manner as to face each other, and each support frame 43 has a vertical slot 43a formed thereon. As a result, locking protrusions (not shown) formed on both ends of the atomization member 30 are fitted to the vertical slots 43a of the support frames 43 of the plate 41, and a lever 45 to which a cam 44 is fitted is located passed through the pair of support frames 43 and the main body 10.

As the cam 44 rotates through the rotation of the lever 45, the roller-shaped atomization member 30 moves upward and downward along the vertical slots 43a, so that the vertical distance between the spray nozzle 20 and the underside surface of the atomization member 30 is adjustable to the range between 10 mm and 100 mm.

As shown in FIG.2a, the atomization member 30 is moved upward by the rotation of the cam 44 according to the rotation of the lever 45 in such a manner as to be spaced apart from the spray nozzle 20 to a maximum extent, so that the degree of atomization becomes decreased and contrarily, the quantity of discharge becomes increased. On the other hand, as shown in FIG.2b, the atomization member 30 is moved downward by the rotation of the cam 44 according to the rotation of the lever 45 in such a manner as to be close to the spray nozzle 20, so that the degree of atomization becomes increased and contrarily, the quantity of discharge becomes decreased.

The spaced distance range and other structures and functions have been mentioned above, and therefore, an explanation on them will be avoided for the brevity of the description.

In this case, the two air spray parts 21a formed on both sides of the liquid spray nozzle 21b formed on the center of the head 21 of the spray nozzle 20 are located in the perpendicular direction to the long length direction of the roller-shaped atomization member 30, and accordingly, the air sprayed from the two air spray parts 21a crossingly collides and is thus mixed with the liquid sprayed from the liquid spray nozzle 21b, thus forming mist. The mist collides in the long length direction of the roller-shaped atomization member 30.

A reference numeral 43b not explained yet indicates a reinforcing rib, and other parts are the same as above. Therefore, an explanation on them will be avoided for the brevity of the description.

On the other hand, FIG.2c shows the operating state of the sprayer 1a as shown in FIGs.2a and 2b, wherein it can be checked that atomized mist 100 is sprayed from the mist discharge port 15a of the main body 10.

A reference numeral 17 not explained yet indicates an intake pipe.

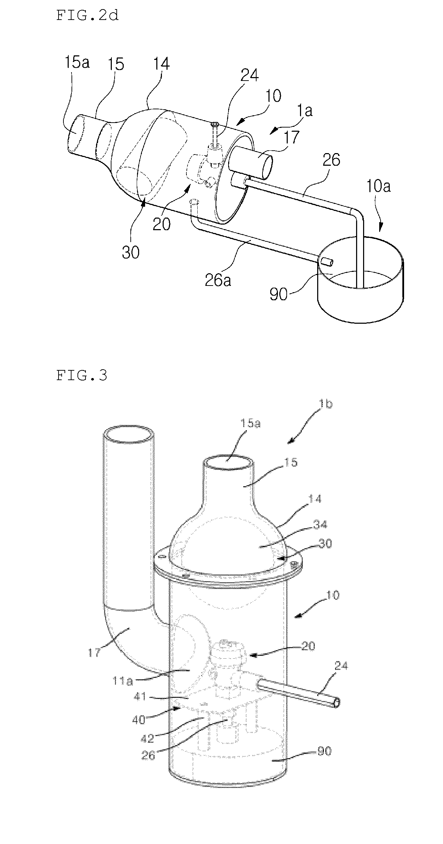

FIG.2d is a perspective view showing the inside of a sprayer 1e as a variation example of the sprayer according to the second embodiment of the present invention. As shown, the atomization member 30 is not adjustable up and down in height, but fixedly located. Further, the liquid 90 is not stored in the main body 10, but the main body 10 is horizontally disposed to the sprayer 1e and has a separate liquid storage vessel 10a connected by means of a liquid pipe 26 thereto and a collection pipe 26a disposed on the bottom portion thereof so as to collect the liquid dropped. Under the above configuration, the spray nozzle 20 is supported directly by means of the bottom surface of the main body 10.

Basically, other parts and configurations are the same as those in FIGS.2a and 2b, and therefore, an explanation on them will be avoided for the brevity of the description. In the drawing, a reference numeral 14 not explained yet indicates a shoulder, 15 indicates a neck, 15a indicates a mist discharge port, and 17 indicates an intake pipe.

FIG.3 is a perspective view showing the inside of the sprayer 1b according to the third embodiment of the present invention. The sprayer 1b has the same configuration as that of FIGS.2a and 2b, except that an atomization member 30 has a spherical shape and is fixedly located, without any adjustment in height, and accordingly, an explanation on the difference will be given for the brevity of the description.

As shown, the atomization member 30 has a spherical shape, and through a plurality of spacers 34, the outer peripheral surface of the upper portion of the atomization member 30 and the inner peripheral surface of a shoulder 14 are spaced preferably 2 to 20 mm apart from each other, more preferably 5 to 15 mm apart from each other, and most preferably 8 to 12 mm apart from each other. Further, the vertical distance between a spray nozzle 20 and the underside surface of the atomization member 30 is preferably in the range between 10 mm and 100 mm, more preferably in the range between 15 mm and 90 mm, and most preferably in the range between 20 mm and 80 mm.

Other parts and configurations are the same as above, and therefore, an explanation on them will be avoided for the brevity of the description.

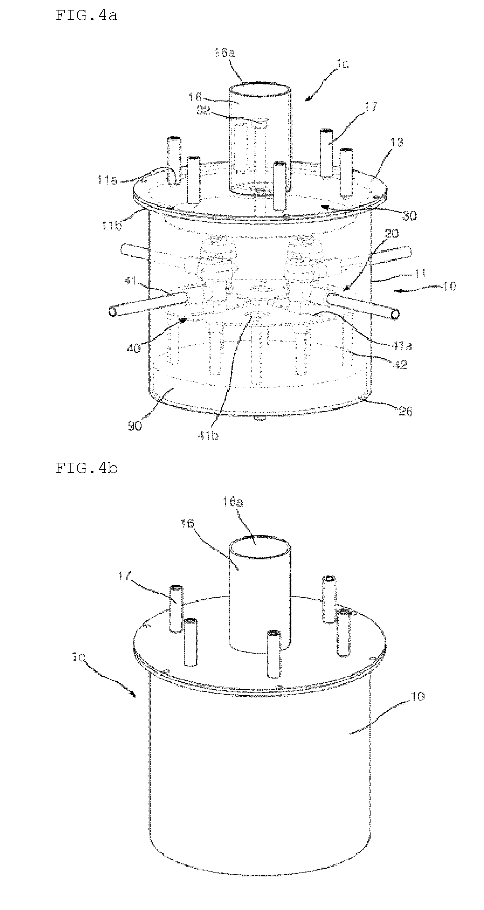

FIG.4a is a perspective view showing the inside of a sprayer 1c according to the fourth embodiment of the present invention. When compared with the sprayer having the round plate-shaped atomization member 30 as shown n FIGS.1b and 1c, the atomization member 30 has a shape of a disc formed convexedly on the upper and lower portions thereof and a plurality of spray nozzles 20 (for example, four spray nozzles as shown) is mounted to obtain a larger quantity of discharge mist than one spray nozzle.

As shown, the atomization member 30 has the shape of the disc formed convexedly on the upper and lower portions thereof, and a mist discharge pipe 16 is located on a top surface plate 13 of a main body 10. The mist discharge pipe 16 has a support rod 16b disposed to traverse the bottom end opening thereof (to which a reference numeral is not designated), and the support rod 16b has a screw hole 16c formed at the center thereof. A height adjustment bolt 32 is passed through the screw hole 16c and coupled to the atomization member 30, so that the vertical distance between the spray nozzle 20 and the convex lower end portion of the atomization member 30 is adjustable to the range between 10 mm and 100 mm.

The atomization member 30 may be fixed to the top surface plate 13 communicating with the mist discharge pipe 16 on which a mist discharge port 16a is formed by means of a plurality of stoppers (see the reference numeral 31 in FIG.1a), without being adjustable in height, which is of course within the spirit of this invention.

In the same manner as above, the outer peripheral surface of the inner peripheral wall of the main body 10 and the outer peripheral surface of the atomization member 30 are spaced preferably 2 to 20 mm apart from each other, more preferably 5 to 15 mm apart from each other, and most preferably 8 to 12 mm apart from each other. Further, the vertical distance between each spray nozzle 20 and the protruded lower portion of the atomization member 30 is preferably in the range between 10 mm and 100 mm, more preferably in the range between 15 mm and 90 mm, and most preferably in the range between 20 mm and 80 mm.

On the other hand, a plurality of intake pipes 17 is formed on the top surface plate 13 of the main body 10, and the spray nozzles (for example, four spray nozzles) are equally spaced apart from each other.

Further, the plurality of spray nozzles 20 is disposed supportedly on a plate 41 having a plurality of support posts 42, and the plate 41 has a plurality of slots 41a formed thereon (to a shape of a cross) toward the center thereof, along which the plurality of spray nozzles 20 can be varied in position.

Furthermore, the plate 41 has a plurality of through holes 41b formed thereon, through which the mist can be gently discharged up and down.

Other parts and configurations are the same as above, and therefore, an explanation on them will be avoided for the brevity of the description.

FIG.4b is a perspective view showing the outer appearance of the sprayer of FIG.4a, wherein the sprayer 1c made of stainless steel has the plurality of intake pipes 17 formed on the top surface of the main body 10 and the mist discharge pipe 16 having the mist discharge port 16a is extended upward from the center thereof.

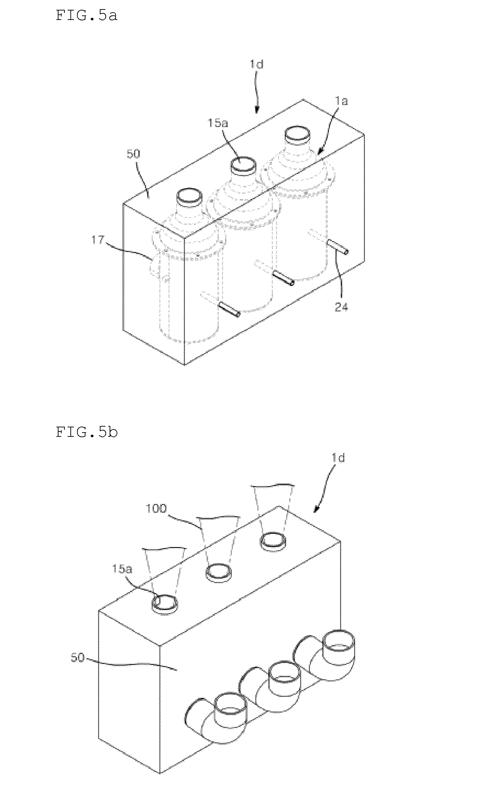

FIG.5a is a perspective view showing the inside of a sprayer 1d according to the fifth embodiment of the present invention, wherein the sprayers 1a parallel to each other according to the second embodiment of the present invention are fixedly accommodated in a box 50. Further, a plurality of intake pipes 17 is formed to communicate with the outside, a plurality of mist discharge ports 15a is formed to communicate with the top surface of the box 50, and a plurality of air pipes 24 is extended outward from the box 50, thus discharging a large quantity of mist.

According to the fifth embodiment of the present invention, any of the sprayers 1, 1a, 1b and 1c as the sprayers parallel to each other can be of course adopted.

FIG.5b shows the operating state of the sprayer 1d of FIG.5a, wherein a large quantity of mist 100 is discharged from the mist discharge ports 15a formed on the top surface of the box 50, and FIG.5c shows the operating state of the sprayer 1d where mist guides 60 for direction conversion are mounted. Further, each mist guide 60 has a guide nozzle 61 mounted on the front end periphery thereof, and FIG.5c shows the operating state wherein only the guide nozzle 61 mounted on the front end periphery of the mist guide 60 located at the center on the top surface of the box 50 is operated.

FIG.6 is a perspective view showing the separated state of a motor 80 and a compressor 70 from the sprayer. The motor 80 and the compressor 70 may be mounted in a single box, while being put therein together with the sprayers 1, 1a, 1b, 1c and 1d, and otherwise, they are separately formed and connected to the sprayer by means of the air pipe (see the reference numeral 24 in FIG.5a). The mounting ways of the motor 80 and the compressor 70 are arbitrarily selected according to the present invention.

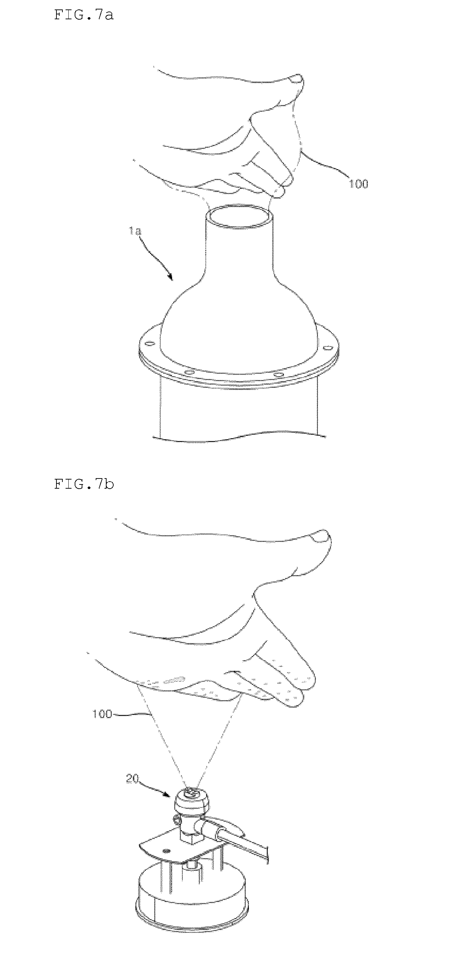

FIGS.7a and 7b are perspective view showing the comparison between micro-particle sizes atomized by the sprayer according to the present invention and one conventional sprayer. In case of the sprayer 1a according to the present invention as shown in FIG.7a, the mist 100 discharged from the mist discharge port 15a has a high degree of atomization so that a user's hand is rarely wet, and contrarily, in case of the conventional sprayer (to which the spray nozzle 20 of the sprayer according to the present invention is adopted) as shown in FIG.7b, the mist sprayed from the sprayer has a low degree of atomization so that the user's hand is instantly wet. Accordingly, it can be appreciated that the formation of the atomization member 30 improves the degree of atomization of the mist sprayed from the sprayer.

### INDUSTRIAL APPLICABILITY

The sprayer of the present invention is capable of effectively atomizing a disinfectant or agricultural chemicals such as an anti-virus agent, an anti-bacterial agent, an anti-fungal agent, a pesticide and the like or an environment improving agent such as a fragrant, a deodorant agent and the like to macro-particle sizes, thus reducing the possibility of the pollution of soil or dwelling environment and conducting the work for the prevention of infectious diseases or for the environment improvement in providing many economical advantages in more environment-friendly manner.

While the present invention has been described in connection with the specific embodiments illustrated in the drawings, they are merely illustrative, and the invention is not limited to these embodiments. It is to be understood that various equivalent modifications and variations of the embodiments can be made by a person having an ordinary skill in the art without departing from the spirit and scope of the present invention. Therefore, the true technical scope of the present invention should not be defined by the above-mentioned embodiments but should be defined by the appended claims and equivalents thereof.

## Claims

1. A sprayer comprising:
a main body where an air intake port and a mist discharge port are formed;
a spray nozzle disposed inside the main body and having an air inlet and a liquid inlet; and
an atomization member adapted to collide the mist sprayed through the spray nozzle thereagainst and to divert the direction of the mist.

2. The sprayer according to claim 1, wherein the atomization member is adjustable in the spaced distance from the spray nozzle so as to control the particle sizes of the mist.

3. The sprayer according to claim 1, wherein the atomization member has a shape of a round plate in such a manner as to be fixed to a top surface plate communicating with a mist discharge pipe on which the mist discharge port is formed by means of a plurality of stoppers, so that the outer peripheral surface of the inner peripheral wall of the main body and the outer peripheral surface of the atomization member are spaced 2 to 20 mm apart from each other and the spaced distances between the spray nozzle and the atomization member and between the atomization member and the top surface plate are in the range between 10 mm and 100 mm.

4. The sprayer according to claim 2, wherein the atomization member has a shape of a round plate and the mist discharge pipe on which the mist discharge port is formed has a support rod disposed to traverse the bottom end opening thereof, the support rod having a screw hole formed at the center thereof, and a height adjustment bolt passed through the screw hole and coupled to the atomization member, so that the vertical distance between the spray nozzle and the atomization member is adjustable to the range between 10 mm and 100 mm.

5. The sprayer according to any one of claims 1 to 4, wherein the spray nozzle is located on a supporter and comprises a head having a liquid spray part and a plurality of air spray parts and a body having the air inlet and the liquid inlet, the supporter being formed of a plate having a plurality of support posts.

6. The sprayer according to claim 1, wherein the main body has a shoulder and a neck having a mist discharge port mounted thereon and the atomization member has a shape of a roller, so that the ratio of the diameter of the main body to the diameter of the roller as the atomization member is in the range between 1 : 0.25 ∼ 0.45, and the vertical distance between the spray nozzle and the underside surface of the atomization member is in the range between 10 mm and 100 mm.

7. The sprayer according to claim 6, wherein the spray nozzle is located on a supporter and comprises a head having a liquid spray part and a plurality of air spray parts and a body having an air inlet and a liquid inlet, the supporter being formed of a plate having a plurality of support posts disposed on the underside thereof, the plate having a pair of support frames erected on the top surface thereof in such a manner as to face each other, each support frame having a vertical slot formed thereon, and wherein a lever to which a cam is fitted is located passed through the pair of support frames and the main body, and as the cam rotates through the rotation of the lever, the roller-shaped atomization member moves upward and downward along the vertical slots, so that the vertical distance between the spray nozzle and the underside surface of the atomization member is adjustable to the range between 10 mm and 100 mm.

8. The sprayer according to claim 7, wherein the plurality of air spray parts of the spray nozzle is located in the perpendicular direction to the long length direction of the roller-shaped atomization member.

9. The sprayer according to claim 1, wherein the main body has a shoulder and a neck having a mist discharge port mounted thereon and the atomization member has a shape of a sphere, so that through a plurality of spacers, the outer peripheral surface of the upper portion of the atomization member and the inner peripheral surface of the shoulder are spaced 2 to 20 mm apart from each other and the vertical distance between the spray nozzle and the underside surface of the atomization member is in the range between 10 mm and 100 mm.

10. The sprayer according to claim 9, wherein the spray nozzle is located on a supporter and comprises a head having a liquid spray part and a plurality of air spray parts and a body having the air inlet and the liquid inlet, the supporter being formed of a plate having a plurality of support posts.

11. The sprayer according to claim 1, wherein the atomization member has a shape of a disc formed convexedly on the upper and lower portions thereof in such a manner as to be fixed to a top surface plate communicating with a mist discharge pipe on which the mist discharge port is formed by means of a plurality of stoppers, so that the outer peripheral surface of the inner peripheral wall of the main body and the outer peripheral surface of the atomization member are spaced 2 to 20 mm apart from each other and the vertical distances between the spray nozzle and the protruded lower surface of the atomization member and between the top surface plate and the protruded upper surface of the atomization member are in the range between 10 mm and 100 mm, wherein a plurality of intake ports is formed on the top surface plate and the spray nozzles are equally spaced apart from each other.

12. The sprayer according to claim 11, wherein the mist discharge pipe has a support rod disposed to traverse the bottom end opening thereof, the support rod having a screw hole formed at the center thereof, and a height adjustment bolt is passed through the screw hole and coupled to the atomization member, so that the vertical distance between the spray nozzle and the protruded lower surface of the atomization member is adjustable to the range between 10 mm and 100 mm.

13. The sprayer according to claim 11 or 12, wherein the spray nozzles are located on the supporter and each comprises a head having a liquid spray part and a plurality of air spray parts and a body having an air inlet and a liquid inlet, the supporter being formed of a plate having a plurality of support posts disposed on the underside thereof, the plate having a plurality of slots formed thereon toward the center thereof, so that the plurality of spray nozzles is varied in position along the plurality of slots.

14. The sprayer according to claim 13, wherein the plate has a plurality of through holes formed thereon.

15. A sprayer comprising:
a plurality of sprayers each having a main body where an air intake port and a mist discharge port are formed, a spray nozzle disposed inside the main body and having an air inlet and a liquid inlet, and an atomization member adapted to collide the mist sprayed through the spray nozzle thereagainst and to divert the direction of the mist, the atomization member being adjustable in the spaced distance from the spray nozzle so as to control the particle sizes of the mist;
a box fixedly containing the plurality of sprayers thereinto; and
a plurality of intake pipes formed to communicate with the outside of the box,
wherein the plurality of mist discharge ports is formed to communicate with the top surface of the box and a plurality of air pipes of the spray nozzles is extended outward from the box.

16. The sprayer according to claim 1 or 15, wherein each mist discharge port has a mist guide mounted thereon.

17. The sprayer according to claim 1 or 2, wherein the main body has a lower portion in which a liquid is stored.

18. The sprayer according to claim 1 or 2, wherein the main body is horizontally disposed to the sprayer and has a separate liquid storage vessel connected by means of a liquid pipe thereto and a collection pipe disposed on the bottom portion thereof so as to collect the liquid dropped, and wherein the spray nozzle is supported directly by means of the bottom surface of the main body.
